# EUROPEAN PATENT APPLICATION

(11) **EP 2 921 125 A1**
(43) Date of publication of application: **23.09.2015**
(21) Application number: 15155833.5
(22) Date of filing: 19.02.2015
(51) Int. Cl.: A61B 18/14

(54) **Electrode assembly for catheter system including struts having a non-uniform thickness**

(30) Priority: 21.03.2014 US 201461968460 P
(71) Applicant: St. Jude Medical, Cardiology Division, Inc., St. Paul MN 55117-9913 (US)
(72) Inventor: Buesseler, Ryan Kenneth, Delano, Minnesota 55328 (US); Dakin, Gregory James, Edina, Minnesota 55424 (US)
(74) Representative: Smaggasgale, Gillian Helen

(57) **Abstract**

Embodiments of the present disclosure provide ablation catheter systems and electrode assemblies and electrode baskets for use in the ablation catheter systems that include one or more struts that have a non-uniform thickness along their length to customize the strut performance. The struts having a non-uniform thickness in accordance with the present disclosure may be utilized in electrode baskets that include any number of struts in any design formation. Common examples include electrode baskets having two, three, four, five or six or more total struts. Suitable struts having a non-uniform thickness along their length may be comprised of a memory shape alloy, such as Nitinol, from a thermoplastic material, or from a combination thereof. Electrode baskets may include thinned struts of different materials.

## Description

### BACKGROUND OF THE DISCLOSURE

### A. CROSS-REFERENCE TO RELATED APPLICATIONS

This application claims the benefit of U.S. Provisional Patent Application No. 61/968,460, filed March 21, 2014, the entire specification of which is incorporated herein.

### B. Field of the disclosure

The present disclosure relates generally to a catheter system for use in a human body, and more particularly to a multi-electrode catheter system, and even more particularly to an electrode assembly for a multi-electrode catheter system including one or more struts that have a non-uniform thickness.

### C. Background Art

Catheter systems are well known in the art for use in medical procedures, such as diagnostic, therapeutic and ablative procedures. Typical catheter systems generally include an elongate catheter extending from a handle. A physician manipulates the catheter through the patient's vasculature to an intended site within the patient. The catheter typically carries one or more working components, such as electrodes or other diagnostic, therapeutic or ablative devices for carrying out the procedures. One or more controls or actuators may be provided on the handle for selectively adjusting one or more characteristics of the working components.

One particular example of a multi-electrode catheter system is an ablative catheter system in which the working component is a multi-electrode component carried at the distal end of a flexible catheter. A control wire extends within the catheter from the multi-electrode component to the handle to operatively connect the multi-electrode component to an actuator on the handle. Manipulating the actuator acts on the control wire to configure the multi-electrode component into a desired configuration for carrying out the ablative procedure. For example, in one such ablative catheter system made by St. Jude Medical, Inc. under the trade name EnligHTN, the multi-electrode component is an electrode assembly in the general form of a basket. Upon locating the electrode basket at a desired location within the patient, manipulating the actuator associated with the handle pulls on the control wire to reconfigure the electrode basket from a collapsed configuration to an expanded configuration in which the electrodes are intended to be in apposition with a surface, such as an arterial wall of the patient. It is thus desirable to provide a highly flexible yet sufficiently strong electrode basket to facilitate apposition of as many of the electrodes of the electrode basket as possible against the arterial wall of the patient when the electrode basket is expanded to achieve optimal performance of the multi-electrode catheter system.

### BRIEF SUMMARY OF THE DISCLOSURE

In one embodiment, the present disclosure is directed to an electrode assembly for an electrode catheter system. The electrode assembly has a longitudinal axis, a proximal end and a distal end, and comprises at least one strut having a length and extending from the proximal end to the distal end of the electrode assembly. The at least one strut has a non-uniform thickness along its length.

In another embodiment, the present disclosure is directed to an electrode assembly for an electrode catheter system. The electrode assembly has a longitudinal axis, a proximal end and a distal end, and comprises a first strut and a second strut. The first strut has a first length and the second strut has a second length. The first strut and the second strut extend from the proximal end to the distal end of the electrode assembly, and the first strut and the second strut have a non-uniform thickness along their lengths.

In yet another embodiment, the present disclosure is directed to an electrode assembly for an electrode catheter system. The electrode assembly has a longitudinal axis, a proximal end and a distal end. The electrode assembly comprises four struts each having a length and extending from the proximal end to the distal end of the electrode assembly. The four struts have a non-uniform thickness about their lengths.

In still another embodiment, the present disclosure is directed to a process for manufacturing an electrode assembly for an electrode catheter system. The process includes preparing a strut for use in the electrode assembly by selectively altering the thickness of the strut at one or more locations along the length of the strut. The selective altering of the thickness along the length of the strut may be completed by selectively grinding, grit blasting, chemically etching, laser ablating, turning, milling, or filing the desired locations along the length to produce a strut having a non-uniform thickness along its length.

The foregoing and other aspects, features, details, utilities and advantages of the present disclosure will be apparent from reading the following description and claims, and from reviewing the accompanying drawings.

### BRIEF DESCRIPTION OF THE DRAWINGS

Figure 1 is a perspective view of one embodiment of a catheter system including a handle, a catheter and an electrode assembly having multiple electrodes, with the electrode assembly being in what is referenced herein as a collapsed configuration.
Figure 2 is a side elevation of the catheter system of Figure 1, with the electrode assembly being in what is referenced herein as an expanded configuration resulting from rotation of a rotatable actuator.
Figure 3 is a perspective view of the electrode assembly of Figure 1 with a plurality of struts carrying the multiple electrodes, the electrode assembly being in its collapsed configuration.
Figure 4 is a perspective view of a strut having a non-uniform thickness along its length wherein the strut has been thinned along its hinges in accordance with one embodiment.
Figure 5 is a perspective view of a strut having a non-uniform thickness along its length wherein the strut has been thinned along its distal leg in accordance with one embodiment.
Figure 6 is a perspective view of a strut having a non-uniform thickness along its length wherein the strut has been thinned along its center segment in accordance with one embodiment.
Figure 7 is a perspective view of a strut having a non-uniform thickness along its length wherein the strut has been thinned along its proximal leg in accordance with one embodiment.
Figure 8 is a perspective view of a strut having a non-uniform thickness along its length wherein the strut has been thinned along its alignment tab in accordance with one embodiment.
Figure 9 is a schematic view of the electrode assembly of Figure 3 at one stage of manufacturing the struts thereof at which point the struts are in the form of a tube, with the tube being in a longitudinally opened and laid flat orientation for illustrative purposes.
Figure 10 is a schematic view of a strut including an oval-shaped cut-out in accordance with one embodiment.
Figure 11 is a schematic view of a strut including a rectangular-shaped cut-out in accordance with one embodiment.
Figure 12 is a schematic view of an expanded strut including an ovate-shaped cut-out in accordance with one embodiment.
Figure 13 is a schematic view of a strut including multiple symmetrical cut-outs in accordance with one embodiment.
Figure 14 is a schematic view of a strut including an elongated cut-out in accordance with one embodiment.

Corresponding reference characters indicate corresponding parts throughout the several views of the drawings.

### DETAILED DESCRIPTION OF THE DISCLOSURE

Embodiments of the present disclosure provide ablation catheter systems and electrode assemblies and electrode baskets for use in the ablation catheter systems that include one or more struts that have a non-uniform thickness along their length to customize the strut performance; that is, one or more portions of the strut have a different wall thickness than one or more other portions of the strut to customize at least the stiffness and/or the bending moment of the resulting strut. The struts having a non-uniform thickness in accordance with the present disclosure may be utilized in electrode baskets that include any number of struts in any design formation. Common examples include electrode baskets having two, three, four, five or six or more total struts. Suitable struts having a non-uniform thickness along their length may be comprised of a memory shape alloy, such as Nitinol, from a thermoplastic material, from a combination thereof, or from any other suitable material. Additionally, electrode baskets including struts of the present disclosure may include struts solely formed a memory shape alloy such as Nitinol, struts solely formed from a thermoplastic material, some struts formed from a memory shape alloy such as Nitinol and some struts formed from a thermoplastic material, or struts formed from any other suitable material. Any or all of the struts may have the same or different thickness profiles, and any or all of the struts may carry one or more electrodes.

In many embodiments, the non-uniform thickness strut will have a reduced thickness at one or more hinge points, at a proximal leg, at an alignment tab, and/or at an electrode position (i.e., at a location where an electrode is positioned on the strut), although thinning of the strut at any location is within the scope of the present disclosure. By varying the thickness of the one or more struts along their length in the electrode assembly, at least the bending moment of inertia of the strut is altered and may be customized to enhance overall device performance as related to radial force, flexibility, stiffness, apposition, and/or tracking, for example. In some embodiments described herein, in addition to the thickness of the strut being controlled and customized at one or more locations along the strut length, the width and/or the length of the strut may also be customized to further control the resulting properties of the strut and the electrode assembly or other device into which it is incorporated. By controlling the width and/or length of the strut, the bending moment may be further controlled and customized to a desired value or range. In one specific embodiment, the electrode basket may include two or more struts having a thinned wall at the electrode position (or other electrode component position) to counter the increased bending moment that the additional electrode component imparts onto the strut. In addition, each strut may optionally have lengths independent of each other in order to impart additional benefits.

In some other embodiments, the thickness of the strut may be reduced along substantial portions of length of the strut, or may even be reduced along the entire length of the strut as compared to conventional struts used in ablation catheter systems. In many embodiments, when the thickness of the strut has been reduced along a substantial portion or even along the entire portion of the length of the strut, the strut may be used in combination with others struts forming the electrode basket that have not been thinned along a substantial portion of the strut.

In many other embodiments, in addition to using variable thickness along the length of the strut to control the bending moment of inertia with or without width and/or length modification, a cut-out or an opening may be introduced into the strut at desired locations to improve resulting performance. By introducing one or more cut-outs or openings into the strut, the resulting device may retain torsional rigidity while having lowered bending forces in and around the location of the cut-out or opening. The cut-out or opening may have any desired geometry that may yield differing operation performance, and may be able to follow the contours of the strut to further improve performance.

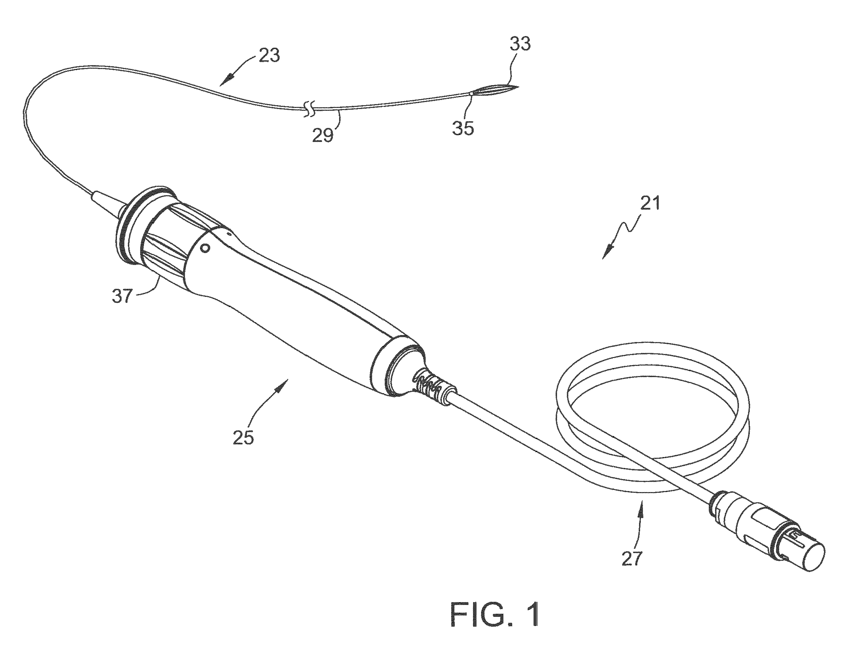

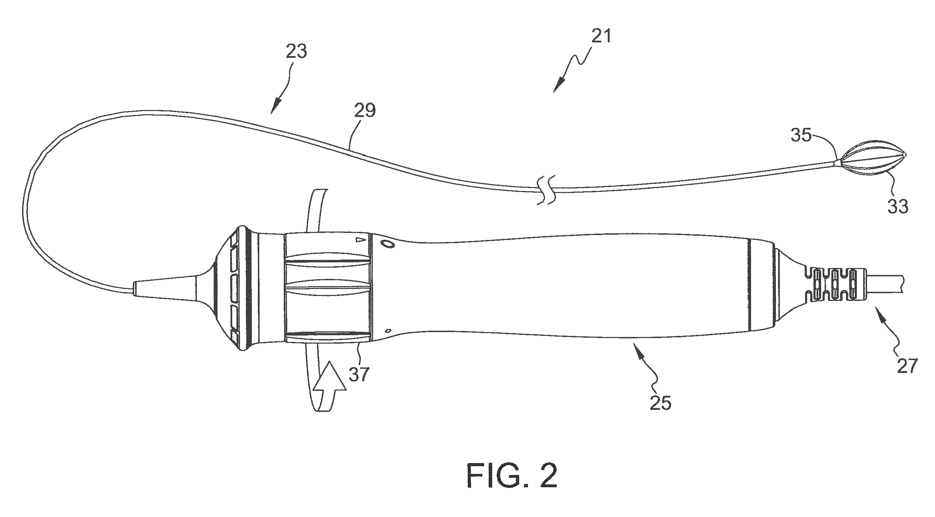

Referring now to the drawings, and in particular to Figures 1 and 2 by way of background and reference, one embodiment of a suitable catheter system 21 into which the novel struts of the present disclosure described hereinbelow may be incorporated in various embodiments includes a flexible catheter 23, a handle 25 to which the catheter is connected, and a conductor assembly 27 for electrically connecting the catheter system to a suitable power supply (not shown). As one example, catheter system 21 illustrated and described herein is suitably constructed for use as an ablation system, such as a renal or heart ablation system. More particularly, catheter system 21 is a multi-electrode renal denervation system. One example of such catheter system 21 is currently made by St. Jude Medical, Inc. under the trade name EnligHTN. General operation of a multi-electrode renal denervation system is known to those of skill in the art and is not described further herein except to the extent necessary to describe the present embodiments of the disclosure. It is also understood that catheter system 21 may be used for any other suitable treatment or purpose without departing from the scope of this disclosure. Additionally, while catheter system 21 is illustrated and described herein as including a flexible catheter 23, catheter system 21 may further include other components used, for example, to guide flexible catheter 23 into a patient - such as, without limitation, a relatively more rigid guide catheter (not shown), or an over-the-wire system (not shown).

Catheter 23 includes an elongate, flexible hollow shaft 29 connected to handle 25 at or near a proximal or rear end of the catheter shaft (not shown because it is hidden by a connector at the front end of the handle 25), and an electrode assembly 33 disposed at or near a distal or front end 35 of the catheter shaft. It is understood, however, that the electrode assembly 33 may be disposed anywhere along the catheter shaft 29 intermediate the proximal end and the distal end 35 thereof without departing from the scope of this disclosure. As used herein, the terms proximal and front, and distal and rear, are used with reference to the orientation of the catheter system 21 illustrated in the various drawings and for the purpose of describing the various embodiments set forth herein, and are not intended as limiting the catheter system and related components to having any particular orientation upon assembly or during operation thereof. In particular, the terms proximal and rear refer to a longitudinal position that is relatively nearer to the handle 25 while the terms distal and front refer to a longitudinal position that is relatively farther from the handle.

The illustrated electrode assembly 33 is in the form of what may be referred to as an electrode basket and is suitably configurable between a collapsed configuration (Figure 1) for maneuvering and positioning the electrode assembly in the patient, and an expanded configuration (Figure 2) for operation of the electrode assembly to perform a desired procedure such as an ablation procedure. An annular (e.g., ringshaped) actuator 37 is mounted on the handle 25 for rotation relative thereto and is operatively connected to the electrode assembly 33 for selectively configuring the electrode assembly between its collapsed and expanded configurations. It is understood that another suitable actuator (e.g., slide, push button, lever, etc.) may be used instead of the rotating actuator 37 to selectively configure the electrode assembly 33 without departing from the scope of this disclosure. In some embodiments, the electrode assembly 33 may be selectively adjustable between an infinite number of configurations (e.g., degrees of expansion) between its collapsed and expanded configurations using the actuator 37.

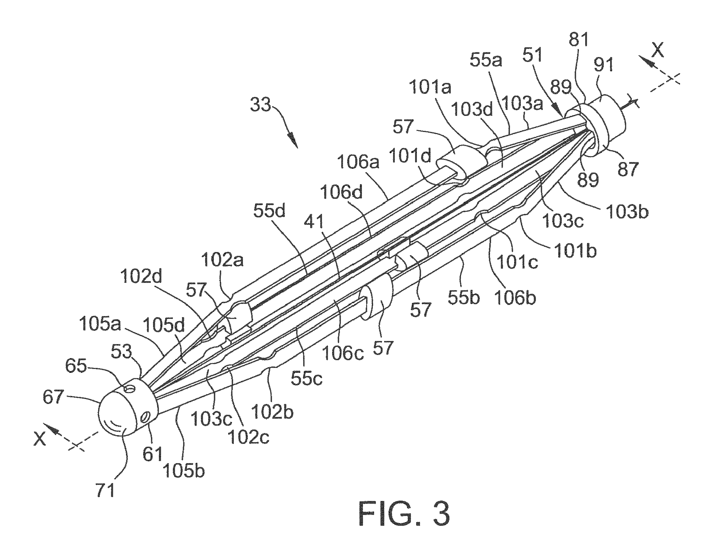

A control line, such as a suitable cable or pull wire 41 (Figure 3) extends from the electrode assembly 33 within the hollow catheter shaft 29 and into the handle 25 for operative connection with the actuator to thereby operatively connect the actuator 37 with the electrode assembly. In some embodiments, two or more pull wires, cables or other suitable control lines may be used for selectively configuring the electrode assembly 33. It is also understood that the control line 41 may be any suitable control line other than a pull wire, such as a cable, string, tie, compression member or other suitable control to operatively connect the electrode assembly 33 to the actuator 37. In other embodiments, any suitable conventional manner for actuating or otherwise selectively configuring the electrode assembly 33 may be used. A suitable twisted electrical wire bundle (not shown) also extends through the hollow catheter shaft 29 from the handle to the electrode assembly to deliver power to the electrode assembly.

With reference now to Figure 3, the electrode assembly 33 has a proximal end 51 at which the assembly is connected to the catheter shaft 29 (e.g., to the distal end 35 of the catheter shaft in the embodiment of Figures 1 and 2), a distal end 53 that in the illustrated embodiment also defines a distal end, or tip, of the catheter 23, and a longitudinal axis X. The illustrated electrode assembly 33 comprises a set of four struts 55a-d, extending coextensively with each other from the proximal end 51 to the distal end 53 of the electrode assembly in circumferentially equal spaced relationship with each other about the longitudinal axis X of the electrode assembly. In other embodiments, the electrode assembly 33 may comprise more or less than four struts 55a-d without departing from the scope of this disclosure. It is also contemplated that the struts 55a-d may be other than equally spaced from each other circumferentially, and/or the struts may be other than coextensive with each other, and remain within the scope of this disclosure.

Each of the struts 55a-d carries at least one electrode 57 disposed at a respective longitudinal position along the strut, i.e., at a respective longitudinal distance along the longitudinal axis X from the proximal end of the electrode assembly. In the embodiment of Figure 3, each of the electrodes 57 is at a different longitudinal position. It is understood that the electrodes 57 may be at longitudinal positions other than those shown in Figure 3. In other embodiments, two, three or all of the electrodes 57 may instead be at the same longitudinal position. It is also understood that multiple electrodes 57 may be carried by any one or all of the struts 55a-d, e.g., with the electrodes on any given strut spaced longitudinally from each other along the strut.

At the distal end 53 of the electrode assembly 33, the struts 55a-d terminate at, and in one embodiment for making the electrode assembly are formed integrally with, a connecting ring 61. In the illustrated embodiment, multiple holes 65 are formed in the sidewall of the connecting ring 61 in spaced relationship with each other about the circumference of the connecting ring. In other embodiments, however, the holes 65 may be omitted. Suitable polymeric sheathing (not shown) may surround the connecting ring 61 to cover the holes 65 following assembly of the electrode assembly 33. A blunt tip 67 includes a rounded head 71.

The control line 41 extends generally along the longitudinal axis X of the electrode assembly 33 through the tip 67 where it is secured to the tip by braising, adhesive, welding, soldering or other suitable securement technique. The holes 65 spaced about the circumference of the connecting ring 61 allow a suitable adhesive to be supplied through the holes for securing the tip 67 on the connecting ring - thereby connecting the distal end 53 of the electrode assembly 33 to the control line 41 for operative connection with the actuator 37 on the handle 25. In other embodiments, the struts 55a-d may be retained at the distal end 53 of the electrode assembly 33 in another suitable manner and remain within the scope of this disclosure. It is also contemplated that the struts 55a-d and connecting ring 61 may be formed separate from each other and subsequently secured together by any suitable securement technique.

At the proximal end 51 of the electrode assembly 33, longitudinal end segments (not shown) of the struts 55a-d are connected to the catheter shaft 29 by a suitable bushing 81. An annular flange 87 extends radially outward from the longitudinally outer end of the bushing 81. The flange 87 has four slots 89 (corresponding to the respective longitudinal end segments 59 of the struts 55a-d) extending longitudinally therethrough radially outward of the bushing 81 and in circumferentially spaced relationship with each other. The longitudinal end segments 59 of the struts 55a-d extend through the respective slots 89 and along the outer surface of the bushing 81.

The bushing 81 (along with the longitudinal end segments 59 of the struts 55a-d) is fitted with a polyimide sleeve 91 filled with suitable adhesive to secure the sleeve and longitudinal end segments of the struts to the bushing. The bushing 81, struts 55a-d and polyimide sleeve 91 are inserted into the distal end 35 of the hollow catheter shaft 29 and secured to the catheter shaft by suitable adhesive to secure the proximal end 51 of the electrode assembly 33 to the distal end of the catheter shaft. It is understood that the struts 55a-d may be connected to the catheter shaft 29 by any other suitable connection that allows the electrode assembly 33 to function in the manner described herein.

The electrode assembly 33 thus has a length defined by the distance along the longitudinal axis X from the proximal end 51 to the distal end 53 of the electrode assembly. To configure the electrode assembly 33 from its collapsed configuration (e.g., as illustrated in Figure 1) to its expanded configuration (e.g., as illustrated in Figure 2), rotation of the actuator 37 relative to the handle 25 operatively pulls on the control wire 41 to thereby pull the tip (i.e., the distal end 53) of the electrode assembly toward the proximal end 51 of the electrode assembly along the longitudinal axis X thereof As the distance between the distal end 53 and the proximal end 51 of the electrode assembly 33 is shortened (i.e., as the length of the electrode assembly decreases), the struts 55a-d are longitudinally compressed and thus forced to bend, or flex transversely outward away from the longitudinal axis X of the electrode assembly to form the expanded configuration of the electrode assembly. As used herein, the expanded configuration of the electrode assembly refers to any transverse movement of the struts 55a-d outward from the collapsed (e.g., initial or pre-set) configuration of the electrode assembly, and may be variably adjusted. Accordingly, it is understood that in the expanded configuration the electrode assembly 33 may be expanded more or less than as illustrated in the various embodiments herein. It is also understood that the collapsed configuration is not intended to mean the most compressed form in which the electrode assembly 33 may be configured, but rather it refers to the relaxed configuration of the electrode assembly free from any external compression forces (such as when compressed to fit the electrode assembly into a guide tube or lumen).

With reference back to Figure 3, each of the struts 55a-d of the illustrated electrode assembly 33 is suitably configured in at least the collapsed configuration of the electrode assembly to have what is referred to herein as a proximal leg 103a-d, a distal leg 105a-d, and a center segment 106a-d extending between and interconnecting the proximal and distal legs of the strut. To facilitate predictable bending of the struts 55a-d, each strut includes a pair of hinges 101a-d, 102a-d in longitudinally spaced relationship with each other, i.e., with one hinge 101a-d intermediate and interconnecting the proximal leg 103a-d and the center segment 106a-d of the strut and the other hinge 102a-d intermediate and interconnecting the distal leg 105a-d and the center segment of the strut. In particular, with reference to the strut 55a in Figure 3, the proximal leg 103a extends from the one hinge 101a to the proximal end of the electrode assembly 33 and the distal leg 105a extends from the other hinge 102a to the connecting ring 61 at the distal end of the electrode assembly.

In the illustrated embodiment, the proximal leg 103a-d and the distal leg 105a-d of each strut 55a-d are of generally equal length. In other embodiments, the proximal leg 103a-d and the distal leg 105a-d may be of unequal length. Also, in the illustrated embodiment, each strut 55a-d has a proximal leg 103a-d, central segment 106a-d and distal leg 105a-d of lengths equal to the proximal leg, central segment and distal leg of each of the other struts so as to maintain symmetry of the electrode assembly 33. It is understood, though, that the respective lengths of the proximal leg 103a-d, center segment 106a-d and distal leg 105a-d of one strut may be different from that of one or more of the other struts. The electrodes 57 are disposed respectively on the center segment 106a-d of each corresponding strut 55a-d.

In the illustrated embodiment of Figure 3, the proximal leg 103a-d, the distal leg 105a-d and the center segment 106a-d each have a uniform width along the respective lengths thereof, i.e., other than where the proximal leg narrows to form the end segments 59 that connect to the bushing 81. However, it is contemplated that in other embodiments the proximal leg 103a-d may have a non-uniform width, such as a width that decreases continuously (i.e., tapers or narrows) from adjacent the hinge 101a-d to the end segment 59. Alternatively, or additionally, the distal legs 105a-d may have a non-uniform width, such as a width that decreases continuously (i.e., tapers, or narrows) from adjacent the hinge 102a-d to adjacent the connecting ring 61. In other embodiments, the width of each proximal leg 103a-d and/or distal leg 105a-d may be tapered in another suitable manner. The width of each center segment 106a-d of each strut 55a-d is generally uniform along its length. Each strut 55a-d of the illustrated embodiment has a narrowed width intermediate the center segment 106a-d and the proximal leg 103a-d to define the hinge 101a-d and another narrowed width intermediate the center segment and the distal leg 105a-d to define the hinge 102a-d. In the illustrated embodiment the width of each strut 55a-d at the hinge 101a-d is equal to the width of the strut at the other hinge 102a-d. However, in other embodiments the width of the strut 55a-d at the hinge 101a-d may be different from the width of the strut at the other hinge 102a-d and remain within the scope of this disclosure.

As used herein, the term "hinge" refers to any suitable intended, preset or predetermined point or zone of flexure or bending in the strut. For example, in the illustrated embodiment of Figure 3, the hinges 101a-d, 102a-d are each formed by generally U-shaped symmetrical cut-outs on opposite sides of each strut 55a-d so that the strut material is continuous across the narrowed width of the strut. The rounded contour of each of the cut-outs reduces the stress at the hinge 101a-d, 102a-d upon bending of the strut 55a-d.

In accordance with the present disclosure, in one embodiment a strut for use in an electrode basket or other device is disclosed that has a non-uniform thickness along its length; that is, the strut includes one or more wall thickness variations along its length. In this embodiment, the strut may have one or more defined points, regions, or areas that have been thinned along the length of the strut to customize and improve the strut's performance when utilized in an electrode basket. By thinning one or more distinct areas of the strut along its length, the bending moment of inertia of the strut is altered in one or more areas thus changing the strut's performance characteristics to allow for the design of the strut to be tailored for specific applications and improved performance. In many embodiments, the strut may be thinned at one or more locations along its length as described herein to allow the electrode basket in which the strut is used to maintain high radial force, yet retain sufficient flexibility for apposition and tracking. Struts prepared according to the present disclosure may be comprised of a memory shape material, such as Nitinol, a thermoplastic material, or a combination of both, and electrode baskets that incorporate the struts may utilize multiple struts of the same or different materials. In some embodiments, all struts present in the electrode basket will be formed from the same material. In other embodiments, two or more materials (e.g., Nitinol and a thermoplastic material) may be used to construct individual struts present in the electrode basket. Some or all of the struts may include one or more electrodes or other electronic devices.

The struts having a non-uniform thickness along their length of the present disclosure may include one or more distinct areas of thinning to improve and/or customize performance of the strut as described herein. In electrode baskets as described above that include multiple struts, for example 2, 3, 4, 5, 6 or more struts, each strut may be identical to the other struts present, or each strut may be different than one or more other struts in the electrode basket; that is, each of the multiple struts may have the same distinct area or areas thinned, or be thinned along the entire length of the strut, or each strut may have different distinct areas thinned. In some embodiments, 2 or more struts are present in the electrode basket and each strut has a similar overall stiffness and has been thinned in identical distinct areas. In some other embodiments, 2 or more struts are present in the electrode basket and each strut has a similar overall stiffness and has been thinned in different distinct areas. In some other embodiments, 2 or more struts are present in the electrode basket and each strut has a similar overall stiffness and some struts have been thinned and other struts have not been thinned. Any or all of the previously described struts may also have width and/or length modifications.

The struts of the present disclosure may be thinned at any point, section, and/or leg, or points, sections, and/or legs along the length of the strut as detailed more fully herein. In one particular embodiment, a strut that includes one or more hinge points may be thinned at some or all hinge points to further performance of the device. In another embodiment, all or a portion of a proximal leg of the strut may be thinned. In another embodiment, all or a portion of a distal leg of the strut may be thinned. In another embodiment, all or a portion of a center segment of the strut may be thinned. In another embodiment, all or a portion of an alignment tab of the strut may be thinned. In another embodiment, all or a portion of the strut where an electrode (or other electrical device such as a thermocouple, etc.) may be positioned may be thinned; that is, the portion of the strut where the electrical component is positioned is thinned such that the electrical component is positioned onto a thinned section of the strut to reduce the overall profile of the strut and hence the electrode basket into which it is incorporated. In another embodiment, the bend points of the strut have been thinned and the remaining portions of the strut have not been thinned. In another embodiment, the bend points of the strut have not been thinned and the remaining portions of the strut have been thinned. Any one or more of these embodiments may be combined together such that a strut may have two or more defined areas thinned in accordance with the present disclosure.

When one or more distinct points, areas, sections or legs of a strut are thinned in accordance with the present disclosure, the resulting thinned portion may have a reduced thickness suitable for the desired embodiment. In some embodiments, the resulting thinned portion may have a reduced thickness of about 1%, or even 2%, or even 5%, or even 7%, or even 10%, or even 15%, or even 20%, or even 25%, or even 30%, or even 40%, or even 45%, or even 50%, or even 60%, or even 70%, or even 80%, or even 85%, or even 90%, or even 95%, as compared to non-thinned areas. Of course, as will be recognized by one skilled in the art based on the disclosure herein, different points, areas, sections or legs of a strut may be thinned a different amount that other points, areas, sections, or legs of the strut in accordance with the present disclosure.

In one specific embodiment, the strut may be thinned along its entire length; that is, the strut is not thinned only at certain points, areas, sections, or legs, but is thinned along its entire, or substantially entire, length. When a strut is thinned along its entire, or substantially entire, length it may be thinned to the amounts noted above. For example, if a conventional strut has a thickness of from about 0.004 inches (about 0.0102 centimeters) to about 0.008 inches (about 0.0203 centimeters), in accordance with the present disclosure it may be thinned along its entire length, or along substantially its entire length, by an amount of about 1%, or even 2%, or even 5%, or even 7%, or even 10%, or even 15%, or even 20%, or even 25%, or even 30%, or even 40%, or even 45%, or even 50%, or even 60%, or even 70%, or even 80%, or even 85%, or even 90%, or even 95%, as compared to its original thickness.

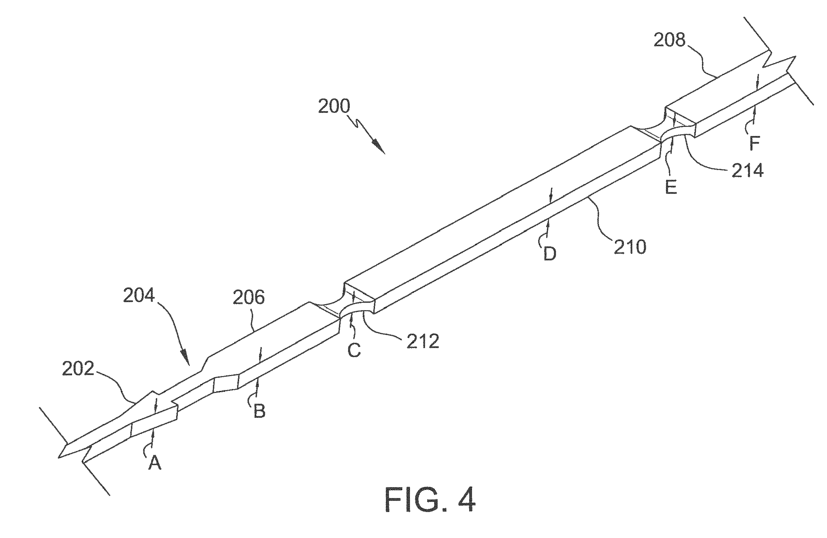

Referring now to Figure 4, there is shown a perspective view of strut 200 in accordance with one embodiment of the present disclosure. Strut 200 includes alignment member 202 having thickness A near proximal end 204 of strut 200, proximal leg 206 having thickness B, distal leg 208 having thickness F, center segment 210 having thickness D and extending between and interconnecting proximal leg 206 and distal leg 208, hinges 212 and 214 having thicknesses C and E respectively and in longitudinally spaced relationship with each other, with one hinge 212 intermediate and interconnecting the proximal leg 206 and the center segment 210 of strut 200 and the other hinge 214 intermediate and interconnecting distal leg 208 and center segment 210 of strut 200. Figure 4 shows proximal leg 206 and distal leg 208 of strut 200 being of generally equal length. In other embodiments, some of which are described herein, proximal leg 206 and the distal leg 208 may be of unequal length. Figure 4 shows hinges 212 and 214 as being thinned in accordance with the present disclosure as compared to the other components of strut 200; that is, hinges 212 and 214 have a reduced wall thickness as compared to alignment member 202, proximal leg 206, distal leg 208, and center segment 210 such that both thickness C and thickness E are less than thickness A, B, D, and F so as to impact the bending moment and stiffness of strut 200. Thickness C and E may be the same or different. Although shown in Figure 4 as gradual thinning into hinges 212 and 214, it is within the scope of the present disclosure to have a more distinct edge where the thinning occurs as opposed to a general thinning arc. Also, although strut 200 is shown in Figure 4 as having both hinges 212 and 214 thinned, it is within the scope of the present disclosure to thin only one of hinges 212 or 214, or to thin one hinge more than the other.

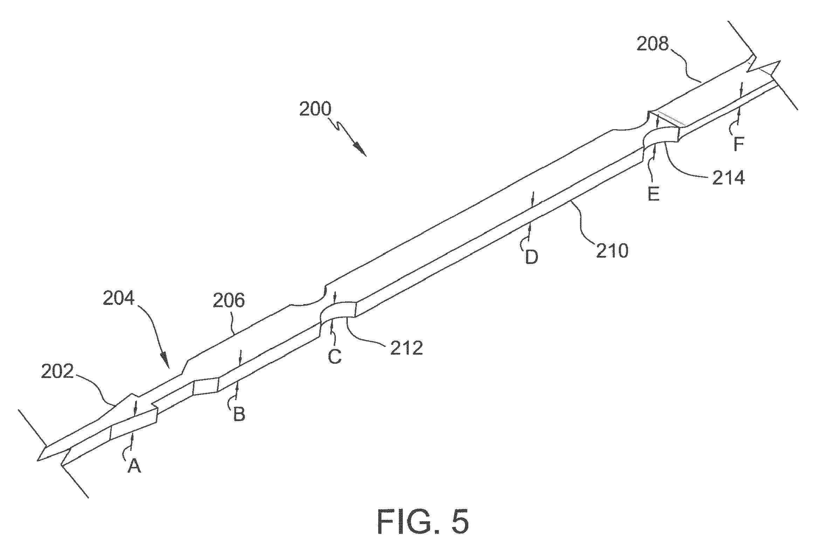

Referring now to Figure 5, there is shown a perspective view of strut 200 in accordance with one embodiment of the present disclosure. Strut 200 includes alignment member 202 having thickness A near proximal end 204 of strut 200, proximal leg 206 having thickness B, distal leg 208 having thickness F, center segment 210 having thickness D and extending between and interconnecting proximal leg 206 and distal leg 208, hinges 212 and 214 having thicknesses C and E respectively and in longitudinally spaced relationship with each other, with one hinge 212 intermediate and interconnecting the proximal leg 206 and the center segment 210 of strut 200 and the other hinge 214 intermediate and interconnecting distal leg 208 and center segment 210 of strut 200. Figure 5 shows proximal leg 206 and distal leg 208 of strut 200 being of generally equal length. In other embodiments, some of which are described herein, proximal leg 206 and the distal leg 208 may be of unequal length. Figure 5 shows distal leg 208 as being thinned in accordance with the present disclosure as compared to the other components of strut 200; that is, distal leg 208 has a reduced thickness as compared to alignment member 202, proximal leg 206, hinges 212 and 214, and center segment 210 such that thickness F is less than thickness A, B, C, D, and E so as to impact the bending moment and stiffness of strut 200. Although shown in Figure 5 as gradual thinning into distal leg 208 it is within the scope of the present disclosure to have a more distinct edge where the thinning occurs as opposed to a general thinning arc.

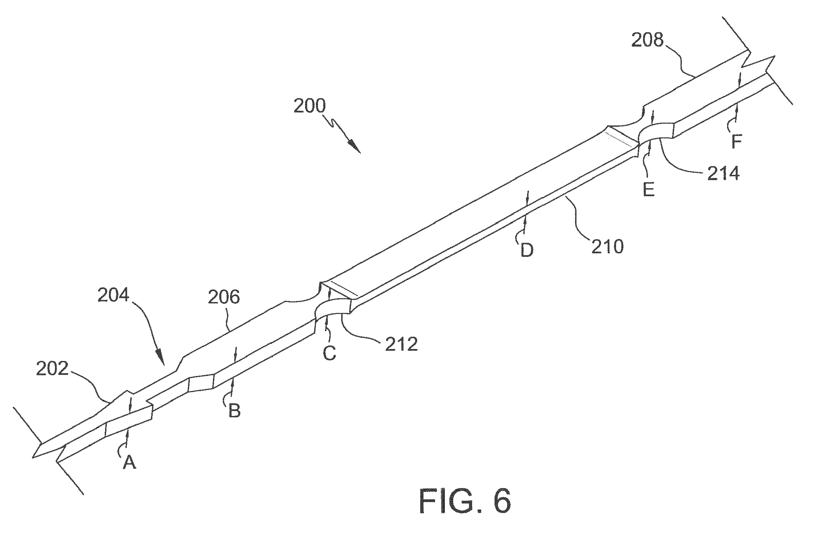

Referring now to Figure 6, there is shown a perspective view of strut 200 in accordance with one embodiment of the present disclosure. Strut 200 includes alignment member 202 having thickness A near proximal end 204 of strut 200, proximal leg 206 having thickness B, distal leg 208 having thickness F, center segment 210 having thickness D and extending between and interconnecting proximal leg 206 and distal leg 208, hinges 212 and 214 having thicknesses C and E respectively and in longitudinally spaced relationship with each other, with one hinge 212 intermediate and interconnecting the proximal leg 206 and the center segment 210 of strut 200 and the other hinge 214 intermediate and interconnecting distal leg 208 and center segment 210 of strut 200. Figure 6 shows proximal leg 206 and distal leg 208 of strut 200 being of generally equal length. In other embodiments, some of which are described herein, proximal leg 206 and the distal leg 208 may be of unequal length. Figure 6 shows center segment 210 as being thinned in accordance with the present disclosure as compared to the other components of strut 200; that is, center segment 210 has a reduced thickness as compared to alignment member 202, distal leg 208, proximal leg 206, and hinges 212 and 214 such that thickness D is less than thickness A, B, C, E, and F so as to impact the bending moment and stiffness of strut 200. Although shown in Figure 6 as gradual thinning into center segment 210 it is within the scope of the present disclosure to have a more distinct edge where the thinning occurs as opposed to a general thinning arc.

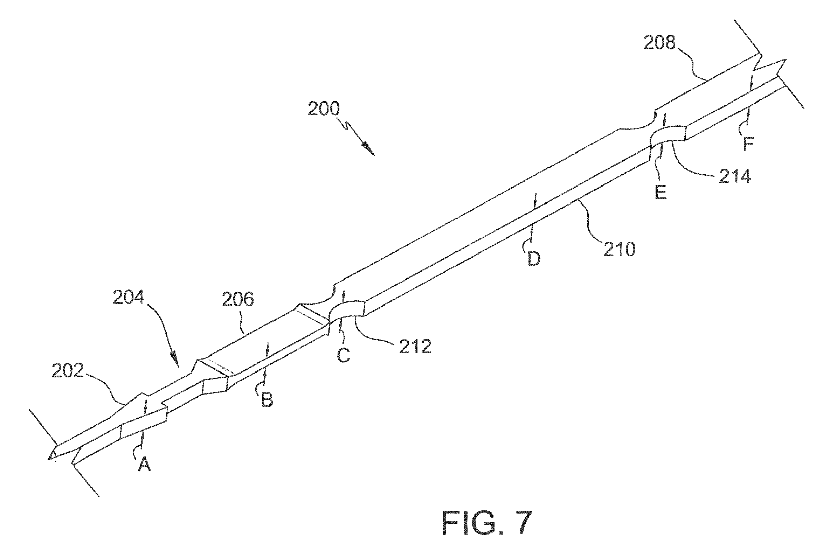

Referring now to Figure 7, there is shown a perspective view of strut 200 in accordance with one embodiment of the present disclosure. Strut 200 includes alignment member 202 having thickness A near proximal end 204 of strut 200, proximal leg 206 having thickness B, distal leg 208 having thickness F, center segment 210 having thickness D and extending between and interconnecting proximal leg 206 and distal leg 208, hinges 212 and 214 having thicknesses C and E respectively and in longitudinally spaced relationship with each other, with one hinge 212 intermediate and interconnecting the proximal leg 206 and the center segment 210 of strut 200 and the other hinge 214 intermediate and interconnecting distal leg 208 and center segment 210 of strut 200. Figure 7 shows proximal leg 206 and distal leg 208 of strut 200 being of generally equal length. In other embodiments, some of which are described herein, proximal leg 206 and the distal leg 208 may be of unequal length. Figure 7 shows proximal leg 206 as being thinned in accordance with the present disclosure as compared to the other components of strut 200; that is, proximal leg 206 has a reduced thickness as compared to alignment member 202, distal leg 208, hinges 212 and 214, and center segment 210 such that thickness B is less than thickness A, C, D, E, and F so as to impact the bending moment and stiffness of strut 200. Although shown in Figure 7 as gradual thinning into proximal leg 206 it is within the scope of the present disclosure to have a more distinct edge where the thinning occurs as opposed to a general thinning arc.

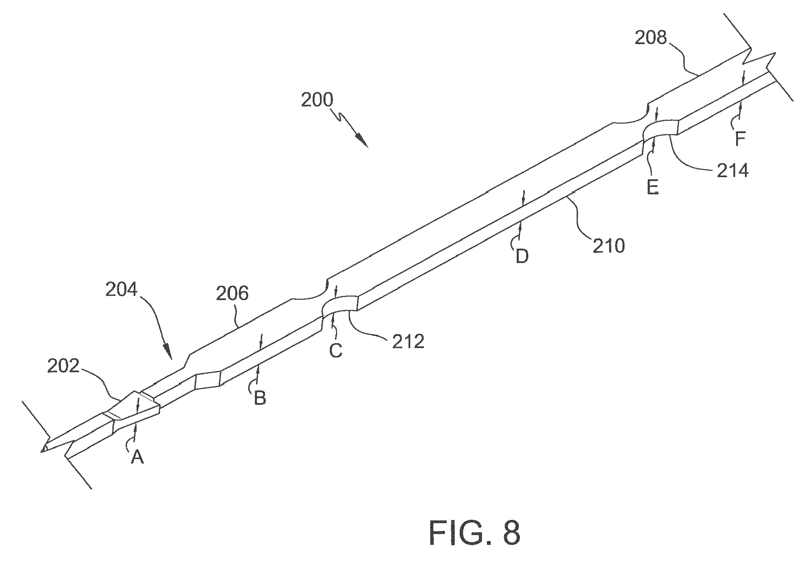

Referring now to Figure 8, there is shown a perspective view of strut 200 in accordance with one embodiment of the present disclosure. Strut 200 includes alignment member 202 having thickness A near proximal end 204 of strut 200, proximal leg 206 having thickness B, distal leg 208 having thickness F, center segment 210 having thickness D and extending between and interconnecting proximal leg 206 and distal leg 208, hinges 212 and 214 having thicknesses C and E respectively and in longitudinally spaced relationship with each other, with one hinge 212 intermediate and interconnecting the proximal leg 206 and the center segment 210 of strut 200 and the other hinge 214 intermediate and interconnecting distal leg 208 and center segment 210 of strut 200. Figure 8 shows proximal leg 206 and distal leg 208 of strut 200 being of generally equal length. In other embodiments, some of which are described herein, proximal leg 206 and the distal leg 208 may be of unequal length. Figure 8 shows alignment member 202 as being thinned in accordance with the present disclosure as compared to the other components of strut 200; that is, alignment member 202 has a reduced thickness as compared to distal leg 208, proximal leg 206, hinges 212 and 214, and center segment 210 such that thickness A is less than thickness B, C, D, E, F so as to allow for a suitable fit with various mating components. Although shown in Figure 8 as gradual thinning into alignment member 202 it is within the scope of the present disclosure to have a more distinct edge where the thinning occurs as opposed to a general thinning arc.

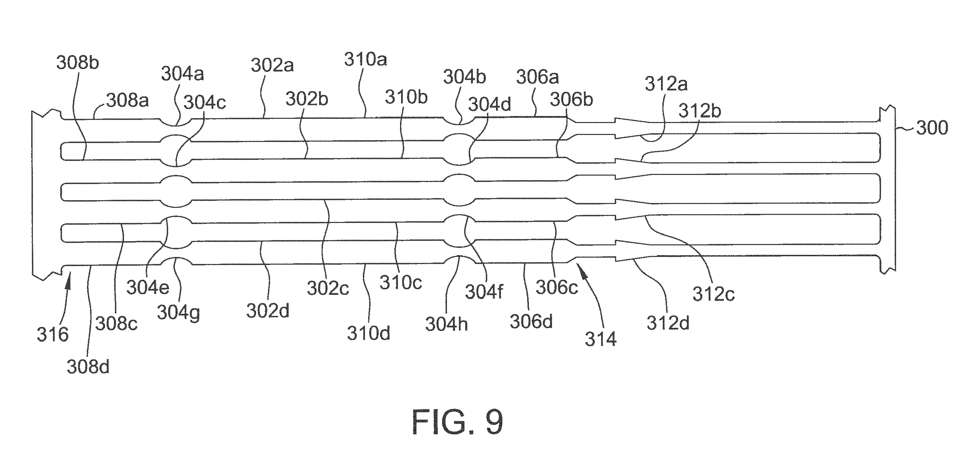

The struts of the present disclosure having a non-uniform thickness along their length for use in a device such as an electrode basket may be fabricated using any number of suitable manufacturing processes to produce the desired non-uniform-thickness configuration as detailed herein. Figure 9 illustrates one suitable embodiment of a method for making struts according to one embodiment of the present disclosure. A unitary tube 300 of a material having a substantially uniform thickness and sufficient strength and shape memory characteristics, such as Nitinol or another memory shape alloy (or a thermoplastic material), is used. The material or materials from which unitary tube 300 is constructed, however, may be any other suitable material and remain within the scope of this disclosure. In other suitable embodiments, a flat sheet of material may be used as a starting material.

In Figure 9, unitary tube 300 is cut lengthwise and laid flat for illustrative purposes. The desired pattern of struts 302a-d is laser cut into unitary tube 300 using a suitable laser process to provide hinges 304a-h, proximal legs 306a-d, distal legs 308a-d, and center segments 310a-d. As illustrated in Figure 9, unitary tube 300 is initially longer than the length of the struts 302a-d. Alignment members 312a-d are formed on each struts 302a-d during the laser cutting process longitudinally outward of the ends of the struts near what eventually becomes the proximal end 314 of struts 302a-d.

Once struts 302a-d are formed in unitary tube 300, an initial slight amount of preset expansion is formed in unitary tube 300 (as illustrated in Figure 3) using an internal and external die assembly or other suitable technique and then heat setting the tube to give unitary tube 300 its collapsed (e.g., initial or preset) configuration. Such preset expansion gives struts 302a-d increased shaped memory and facilitates more predictable bending of struts 302a-d into the desired expanded configuration of the electrode assembly. Following the heat setting, unitary tube 300 is cut adjacent alignment members 312a-d to define the proximal legs 306a-d of struts 302a-d for connecting struts 302a-d to a bushing (not shown) and subsequently to catheter shaft (not shown) in a manner known in the art. A tip (not shown) is secured to distal end 316 of struts 302a-d in a manner known in the art.

Once the struts have been formed and optionally separated, one or more desired points, areas or locations of the struts may be thinned according to the present disclosure to impact at least the bending performance and stiffness of the strut and the resulting device into which it is incorporated, which may suitably be an electrode assembly. Of course, as one skilled in the art would recognize based on the disclosure herein, the timing of the thinning is not generally critical and the tube wall could be thinned at one or more specific locations prior to the laser cutting noted above, or alternatively one or more of the struts could be thinned prior to the strut separation within the scope of the present disclosure.

Any suitable thinning technique for material removal may be used to thin the desired portion or portions of the strut, including for example, grinding, grit blasting, chemical etching/masking, partial laser ablation, turning, milling, filling, and the like and combinations thereof.

In other embodiments of the present disclosure, struts having a non-uniform thickness along their length for use in an electrode basket may further be modified by varying the width of the strut at one or more locations thereon to further customize the performance of the strut. Further in other embodiments, when two or more struts are present in an electrode basket or other device, the two or more struts may be the same or different and may include one or more points of non-uniform thickness, one or more points of varying width, and may also have lengths independent of each other in order to maximize performance characteristics such as radial force, apposition, and/or track force.

In other embodiments of the present disclosure, struts having a non-uniform thickness along their length (and optionally differing widths and lengths as described herein) may additionally include one or more interior openings (or cut-outs) disposed between the side edges of the strut (i.e., generally in the middle of the strut). By introducing an opening into the center of the strut, the strut (and hence the electrode basket or other device into which it is incorporated) retains torsional rigidity while having lowered bending forces around where the opening is located. The opening may be on a section of the strut that has been thinned, or may be on a section of the strut that has not been thinned, or a combination thereof One or more openings may be present on a thinned strut, optionally including varied widths and lengths. The opening may be a simple circular or oval hole, or it may be any of various geometries which could yield differing operating performance. Any such opening may be customized to follow the contours of the strut and the resulting device into which the strut is incorporated, either through a single, simple bend, or a tortuous design.

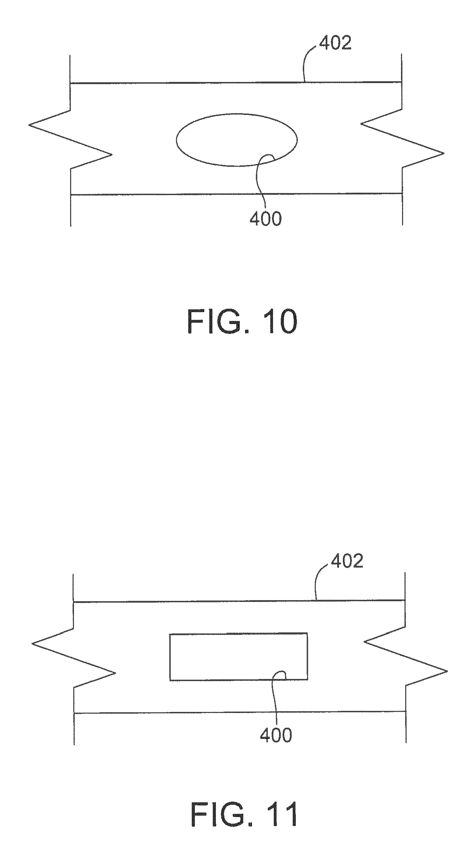

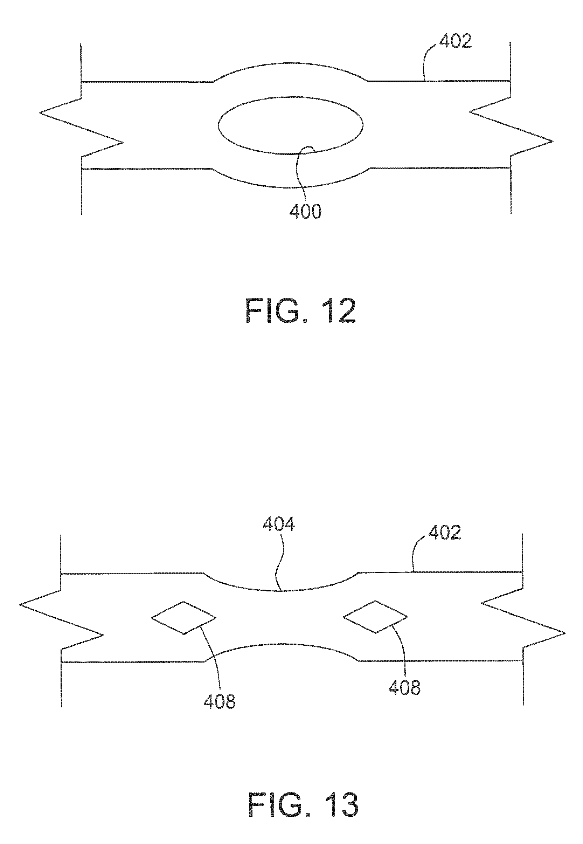

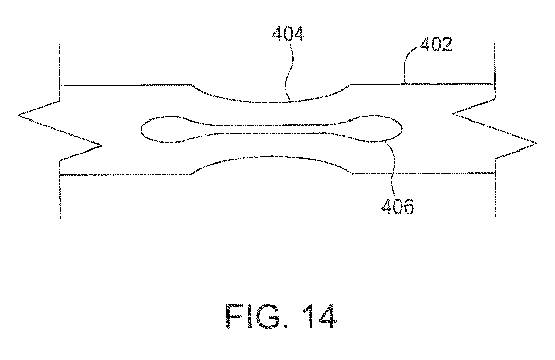

As noted, an opening 400 on a strut 402 may be circular or oval (Figure 10), rectangular (Figure 11) or other suitable shaped as desired for an embodiment. In an alternative embodiment of Figure 12, strut 402 is widened at a desired location (such as at a hinge) to accommodate an opening 400 having a generally ovate shape. Figure 13 illustrates another alternative embodiment in which a hinge 404 of strut 402 includes opposed, symmetrical openings 408 of generally diamond shapes. In Figure 14 a single elongated opening 406 extends lengthwise along strut 402. It is contemplated that in other embodiments the any opposed openings may not be symmetrical.

Although certain embodiments of this disclosure have been described above with a certain degree of particularity, those skilled in the art could make numerous alterations to the disclosed embodiments without departing from the spirit or scope of this disclosure. All directional references (e.g., upper, lower, upward, downward, left, right, leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of the disclosure. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting. Changes in detail or structure may be made without departing from the spirit of the disclosure as defined in the appended claims.

When introducing elements of the present disclosure or the preferred embodiment(s) thereof, the articles "a", "an", "the", and "said" are intended to mean that there are one or more of the elements. The terms "comprising", "including", and "having" are intended to be inclusive and mean that there may be additional elements other than the listed elements.

Reference throughout the specification to "various embodiments," "some embodiments," "one embodiment," "an embodiment," or the like, means that a particular feature, structure, or characteristic described in connection with the embodiment is included in at least one embodiment. Thus, appearances of the phrases "in various embodiments," "in some embodiments > " "in one embodiment," "in an embodiment," or the like, in places throughout the specification are not necessarily all referring to the same embodiment. Furthermore, the particular features, structures, or characteristics may be combined in any suitable manner in one or more embodiments. Thus, the particular features, structures, or characteristics illustrated or described in connection with one embodiment may be combined, in whole or in part, with the features structures, or characteristics of one or more other embodiments without limitation.

It will be appreciated that the terms "proximal" and "distal" may be used throughout the specification with reference to a clinician manipulating one end of an instrument used to treat a patient. The term "proximal" refers to the portion of the instrument closest to the clinician and the term "distal" refers to the portion located furthest from the clinician. It will be further appreciated that for conciseness and clarity, spatial terms such as "vertical," "horizontal," "up," and "down" may be used herein with respect to the illustrated embodiments. However, surgical instruments may be used in many orientations and positions, and these terms are not intended to be limiting and absolute.

## Claims

1. An electrode assembly for an electrode catheter system, the electrode assembly having a longitudinal axis, a proximal end and a distal end, the electrode assembly comprising at least one strut having a length and extending from the proximal end to the distal end of the electrode assembly, the at least one strut having a non-uniform thickness along its length.

2. The electrode assembly of claim 1 wherein the at least one strut includes at least one hinge point, wherein the at least one hinge point has a thickness different than the thickness of the remainder of the at least one strut.

3. The electrode assembly of claim 1 or 2 wherein the at least one strut includes a proximal tail, wherein the proximal tail has a thickness different than the thickness of the remainder of the at least one strut.

4. The electrode assembly of any one of claims 1 to 3 wherein the at least one strut includes a bushing tab, wherein the bushing tab has a thickness different than that thickness of the remainder of the at least one strut.

5. The electrode assembly of any one of claims 1 to 4 wherein the at least one strut is constructed from a material selected from the group consisting of a memory shape material, a thermoplastic material, and a combination thereof

6. The electrode assembly of any one of claims 1 to 5 wherein the at least one strut is constructed of Nitinol.

7. The electrode assembly of any one of claims 1 to 6 wherein the at least one strut includes at least one cutout.

8. An electrode assembly for an electrode catheter system, the electrode assembly having a longitudinal axis, a proximal end and a distal end, the electrode assembly comprising a first strut and a second strut, the first strut having a first length and second strut having a second length, wherein the first strut and the second strut extend from the proximal end to the distal end of the electrode assembly, and wherein the first strut and the second strut have a non-uniform thickness along their lengths.

9. The electrode assembly of claim 8 wherein the first strut has a width profile different that the width profile of the second strut.

10. The electrode assembly of claim 8 or 9 wherein at least one of the first strut or second strut includes at least one cutout.

11. The electrode assembly of any one of claims 8 to 10 wherein at least one of the first strut or second strut includes at least one hinge point.

12. The electrode assembly of any one of claims 8 to 11 wherein the first strut is constructed from a material selected from the group consisting of a memory shape material, a thermoplastic material, and a combination thereof and the second strut is constructed from a material selected from the group consisting of a memory shape material, a thermoplastic material, and a combination thereof.

13. An electrode assembly for an electrode catheter system, the electrode assembly having a longitudinal axis, a proximal end and a distal end, the electrode assembly comprising four struts each having a length and extending from the proximal end to the distal end of the electrode assembly, and wherein each of the four struts have a non-uniform thickness about their lengths.

14. The electrode assembly of claim 13 wherein one or more of the four struts includes at least one electrode.

15. The electrode assembly of claim 13 or 14 wherein each of the four struts includes at least one hinge point.
